# EUROPEAN PATENT SPECIFICATION

(11) **EP 1 195 145 B1**
(45) Date of publication and mention of the grant of the patent: **17.05.2006**
(21) Application number: 01830585.4
(22) Date of filing: 14.09.2001
(51) Int. Cl.: A61C 1/00, A61L 2/18

(54) **A water line for supplying sterilising and sterile fluids in dental equipment**
Wasserleitung zur Zufuhr von sterilisierenden und sterilen Flüssigkeiten in einer zahnärztlichen Anlage
Conduite d'eau pour l'apport de fluides de stérilisation et stériles dans un équipement dentaire

(30) Priority: 19.09.2000 IT BO200542
(43) Date of publication of application: 10.04.2002
(73) Proprietor: CASTELLINI S.p.A., I-40013 Castel Maggiore (Bologna) (IT)
(72) Inventor: Castellini, Franco, 40124 Bologna (IT)
(74) Representative: Lanzoni, Luciano

(56) References cited:
- EP-A- 0 734 692
- EP-A- 1 161 959
- WO-A-95/20366
- US-A- 5 147 613
- US-A- 5 526 841

## Description

The present invention relates to a water line for supplying sterilising fluids and sterile user fluids in dental equipment such as dental units, as disclosed in for example WO-A-95 20366

At present, the water line of a typical dental unit comprises a main branch that supplies a fluid (for example, water from the mains) to the handpieces used by the health-care provider.

This branch is connected to secondary branches for supplying fluids to a spittoon and to a tumbler, whilst other sub-branches have been added to supply the main line and, in some cases, the secondary lines, with fluids for disinfecting or sterilising the line (starting upstream of the line) and with sterile fluids to be used by the health-care provider during a treatment session.

Each of the sub-branches, of which there are usually two, to supply the disinfectant or sterilising fluids and the sterile fluid is equipped, at the upstream end of it, with detachable means for attaching it to a container containing the fluid to be used. These means allow the container to be quickly and easily changed with another containing the same or different fluid, depending on the requirements of treatment.

The attachment means comprise a cap-like device which locks on to the top of the container (by means of a screw or snap-on fitting) and which is connected directly to the conduit that forms the sub-branch.

Each cap has a tube which, when the container is attached to it, is located inside the container in such a way that the fluid it contains can be sucked into the sub-branch automatically by the dental unit control program (disinfection or sterilising cycles) or manually by the health-care provider depending on specific requirements using appropriate valves or other customary controls on the dental unit.

The current structuring of the sub-branches has the disadvantage of not allowing the sub-branch for the sterile liquid to be sterilised, since the two branches are separate and are connected to the main conduit at two separate points.

As a result of this sub-branch structure, the sub-branch that supplies the sterile liquid cannot be sterilised and/or disinfected. Over time, this sub-branch can become contaminated (for example, by agents from the outside when no fluid container is fitted). From here, the contaminating agents can spread to the main conduit, greatly diminishing the effectiveness of sterilising and/or disinfection processes.

The aim of the present invention is to overcome the above mentioned disadvantage by providing a water line for supplying disinfectant/sterilising fluids and sterile user fluids in a dental unit and capable, without excessively varying the structure of the water line, of maintaining every conduit and branch forming part of the line at the highest possible level of sterility.

This aim is substantially achieved by a water line supplying sterilising fluids and sterile user fluids in dental equipment comprising a main conduit supplying a mains fluid to a plurality of branches connecting with corresponding dental handpieces; the main conduit being connected, upstream of the connecting branches, to a first sub-branch that delivers sterile user fluid instead of the mains fluid, and to a second sub-branch that delivers a disinfectant/sterilising fluid to the main conduit and to the connecting branches; the first and second sub-branches being each equipped with an element or capsule used to attach a container containing the fluids to be supplied and a tube connected to the first or second sub-branch, insertable in the container and designed to suck in fluid on a command from related control means; the second sub-branch which supplies the disinfectant/sterilising fluid starting at the corresponding capsule and connecting directly with the first sub-branch so as to allow the control means to alternately supply the disinfectant/sterilising fluid and the sterile fluid along a single first sub-branch connected to the main conduit.

The technical features of the present invention, in accordance with the above-mentioned aims, are set out in the claims below and the advantages more clearly illustrated in the detailed description which follows, with reference to the accompanying drawings, which illustrate a preferred embodiment of the invention without restricting the scope of the inventive concept, and in which:
- Figure 1 is a diagram of a part of the water line of a dental unit equipped with a water line for supplying sterilising and sterile user fluids according to the present invention;
- Figure 2 is a scaled-up schematic detail view of the water line illustrated in Figure 1.

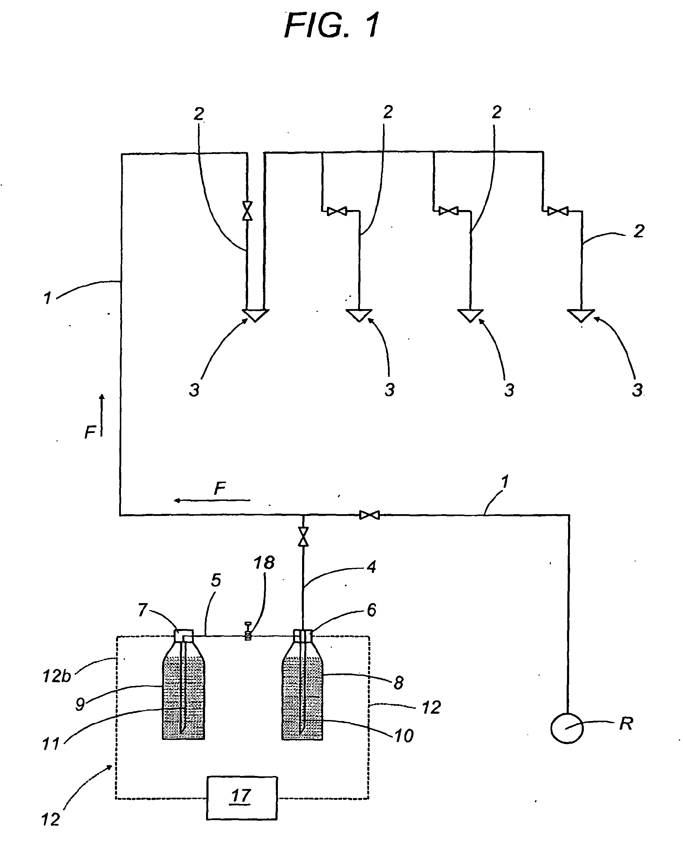

With reference to the accompanying drawings, in particular, Figure 1, the water line according to the present invention is designed to supply disinfectant / sterilising fluids and sterile user fluids in dental equipment such as dental units.

This water line essentially comprises a main conduit 1 that supplies a mains fluid (usually water) to a plurality of branches 2 connecting with corresponding dental handpieces 3. The branches 2 vary in number according to the number of handpieces 3 on the dental unit. In the embodiment described here purely by way of example, there are four handpieces.

Upstream of the connecting branches 2 relative to the direction of fluid flow, indicated by the arrow F, the main conduit 1 is connected to a first sub-branch 4 that delivers the sterile fluid instead of the mains fluid, and a second sub-branch 5 that delivers the fluid for disinfecting / sterilising the main conduit 1 and the connecting branches 2.

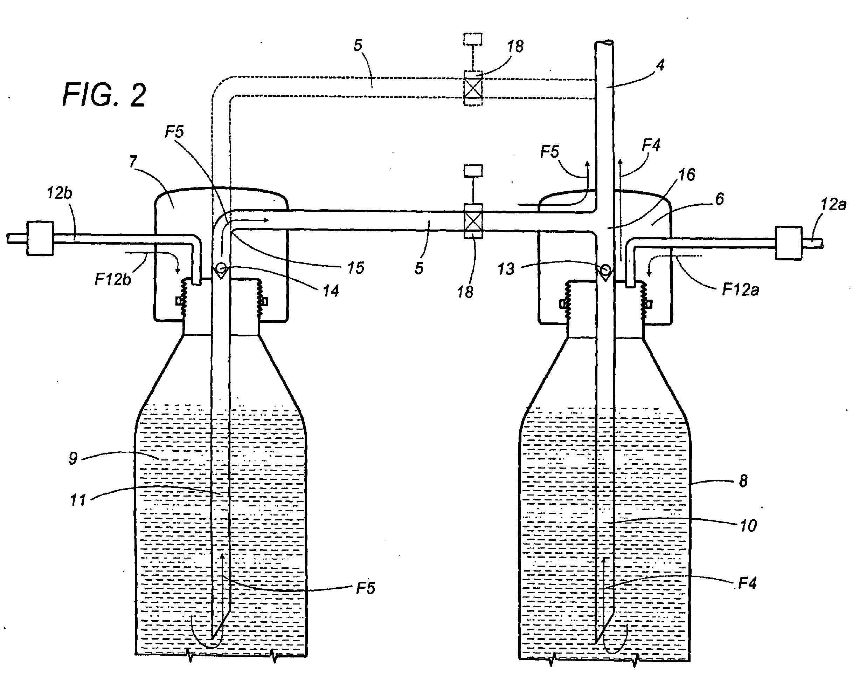

As also shown in Figure 2, the first and second sub-branches 4 and 5 are each equipped with an element or capsule 6, 7 for attaching them (by a screw-on or snap-on fitting) to corresponding containers 8, 9 containing the fluids to be supplied, and a tube 10, 11, connected to the corresponding first or second sub-branch 4 or 5 and insertable in the container 8 or 9 when the latter is attached to the capsule 6, 7.

The tubes 10 and 11 suck in the fluids on a command from related control means 12 consisting, for example, of a pneumatic system (present on the entire dental unit but illustrated only schematically as two conduits 12a and 12b and a control unit 17) and connected to the containers 8 and 9 to create a pressure inside them such as to draw the fluids first into the sub-branches 4 and 5 and then into the main conduit 1.

As clearly shown in Figures 1 and 2, the second sub-branch 5 which supplies the disinfectant /sterilising fluid starts at the corresponding capsule 7 and connects directly with the first sub-branch 4 which supplies the sterile fluid so as to allow the control means 12 to alternately supply the disinfectant/sterilising fluid and the sterile fluid along a single first sub-branch 4 connected to the main conduit 1 (embodiment illustrated by the dashed line in Figure 2).

In a preferred embodiment, the second sub-branch 5 is diverted and joined to the first sub-branch 4 inside the capsule 6 itself, where the sub-branch 4 itself starts, just downstream of the corresponding tube 10.

To achieve operating safety, ensuring that the two fluids do not interfere with each other, each capsule 6 and 7 is equipped, at the top end of the tubes 10 and 11, with a valve 13 and 14 acting in combination with the control means 12 to allow the fluids to flow into the single sub-branch 4 only one at a time, when required.

In other terms, each capsule 6 and 7 is equipped with a non-return valve 13, 14 located at the top end of the corresponding tube 10, 11.

Looking in more detail, the capsule 7 attaching to the second sub-branch 5 to supply the disinfectant/sterilising fluid may be equipped with an upturned "L" duct 15 connected to the second sub-branch 5 to divert the fluid towards the other capsule 6.

The capsule 6 attaching to the first sub-branch 4 to supply the sterile user fluid may be equipped with a "T" duct 16 to allow the fluids to flow into the main conduit 1 alternately.

To further increase the safety of the water system, a second shutoff valve 18 may be fitted to the second sub-branch 5 connecting the two capsules 6, 7 in order to close the second sub-branch 5 and to keep the second sub-branch 5 isolated while the sterile fluid is being used.

In practice, a water line made as described above works in the following manner.

When the line has to be disinfected / sterilised (see Figure 2) the pneumatic conduit 12b is activated (see arrow F12b) so as to allow fluid to flow into the second sub-branch 5 (see arrow F5, with the valve 18 in the open position) and from there into the first sub-branch 4 through the connection made in the capsule 6 in such a way as to flow into the main conduit 1 and into the connecting branches.

Instead, when the sterile fluid has to be used, the pneumatic conduit 12a is activated (see arrow F12a drawn with a dashed line) to allow the sterile fluid to flow into the first sub-branch 4 (see arrow F4 drawn with a dashed line) and from there into the main conduit 1, while the safety valve 18 is kept in the closed position to prevent the two fluids from mixing.

Obviously, after a disinfecting or sterilising cycle, the sub-branch 4 is flushed automatically by the sterile fluid itself, according to the programmed disinfecting / sterilising cycle, the program usually allowing the user to select the fluid used to flush the line once the latter has been disinfected or sterilised.

The water line according to the invention therefore fully achieves the above mentioned aims thanks to its simple, safe structure designed in such a way as to allow a single sub-branch to supply more than one fluid, while keeping this sub-branch at high level of sterility and without drastically modifying the customary structure of that part of a dental unit.

The invention, therefore, not only simplifies the structure of the water line but also enables all the conduits and branches forming part of the water line that supplies the branches 2 to be completely sterilised: in short, the entire water line, from the supply sources to the working branches 2 can be kept in a condition of complete sterility.

## Claims

1. A water line for supplying sterilising fluids and sterile user fluids in dental equipment, the water line comprising a main conduit (1) supplying a mains fluid to a plurality of branches (2) connecting with corresponding dental handpieces (3); the main conduit (1) being connected, upstream of the connecting branches (2), at least to a first sub-branch (4) that delivers sterile user fluid instead of the mains fluid, and a second sub-branch (5) that delivers a disinfectant/sterilising fluid to the main conduit (1) and to the connecting branches (2); the first and second sub-branches (4, 5) being each equipped with an element or capsule (6, 7) used to attach a container (8, 9) containing the fluids to be supplied and a tube (10, 11), connected to the first or second sub-branch (4, 5), insertable in the container (8, 9), and designed to suck in fluid on a command from related control means (12); said water line being **characterised in that** the second sub-branch (5) which supplies the disinfectant/sterilising fluid starts at the corresponding capsule (7) and connects directly with the first sub-branch (4) which supplies the sterile fluid immediately downstream of the corresponding tube (10), in such a way as to allow the control means (12) to alternately supply the disinfectant/sterilising fluid and the sterile fluid along a single first sub-branch (4) connected to the main conduit (1).

2. The water line according to claim 1, **characterised in that** the second sub-branch (5) starts at the corresponding capsule (7) and connects directly with the capsule (6) of the first sub-branch (4) which supplies the sterile fluid.

3. The water line according to claim 1 or 2, **characterised in that** each capsule (6, 7) is equipped, at the top end of the tube (10, 11), with a valve (13, 14) acting in combination with the control means (12) to allow the fluids to flow into the single sub-branch (4) only one at a time, when required.

4. The water line according to claim 1 or 2, **characterised in that** the capsule (7) attaching to the second sub-branch (5) to supply the disinfectant /sterilising fluid is equipped with an upturned "L" duct (15) connected to the second sub-branch (5) to divert the fluid towards the other capsule (6).

5. The water line according to claim 2, **characterised in that** the capsule (6) attaching to the first sub-branch (4) to supply the sterile user fluid is equipped with a "T" duct (16) to allow the fluids to flow into the main conduit (1) alternately.

6. The water line according to claim 3, **characterised in that** each capsule (6, 7) is equipped with a non-return valve (13, 14) located at the top end of the corresponding tube (10, 11).

7. The water line according to claim 1, **characterised in that** the means (12) for controlling the supply of each fluid comprise an air duct (12a, 12b) made in each capsule (6, 7) and designed to be activated by a unit (17) for generating pressure inside the respective container (8, 9) in such a way as to allow the selected fluid to be supplied.

8. The water line according to claim 1, **characterised in that** the second sub-branch (5) connecting the two capsules (6, 7) is equipped with a second shutoff valve (18) designed to close the second sub-branch (5) while the sterile fluid is being used.

## Patentansprüche

1. Wasserleitung zur Zufuhr von sterilisierenden Flüssigkeiten und sterilen Verbraucherflüssigkeiten in einer zahnärztlichen Anlage, wobei die Wasserleitung eine Hauptleitung (1) enthält, die eine Hauptflüssigkeit an eine Anzahl von Zweigleitungen (2) führt, welche mit den entsprechenden zahnärztlichen Handstücken (3) verbunden sind; wobei die Hauptleitung (1) stromaufwärts der verbindenden Zweigleitungen (2) an wenigstens eine erste Unterleitung (4) angeschlossen ist, welche eine sterile Verbraucherflüssigkeit anstelle der Hauptflüssigkeit zuführt, und an eine zweite Unterleitung (5), welche eine desinfizierende/sterilisierende Flüssigkeit an die Hauptleitung (1) und die verbindenden Zweigleitungen (2) führt; wobei die ersten und zweiten Unterleitungen (4, 5) jede mit einem Element oder einer Kapsel (6, 7) versehen sind, dazu benutzt, um einen die zuzuführenden Flüssigkeiten enthaltenden Behälter (8, 9) anzuschliessen, sowie mit einer Kanüle (10, 11), die an die erste oder zweite Unterleitung (4, 5) angeschlossen ist, einsetzbar in den Behälter (8, 9), und die dazu bestimmt ist, auf einen von entsprechenden Steuermitteln (12) kommenden Befehl hin Flüssigkeit anzusaugen; wobei die genannte Wasserleitung **dadurch gekennzeichnet ist, dass** die zweite Unterleitung (5), welche die desinfizierende/sterilisierende Flüssigkeit zuführt, von der entsprechenden Kapsel (7) ausgeht und sich direkt mit der ersten Unterleitung (4) verbindet, welche die sterile Flüssigkeit unmittelbar stromabwärts der entsprechenden Kanüle (10) auf solche Weise zuführt, dass es den Steuermitteln (12) ermöglicht wird, abwechselnd die desinfizierende/sterilisierende Flüssigkeit und die sterile Flüssigkeit entlang einer einzigen ersten Unterleitung (4) zuzuführen, welche an die Hauptleitung (1) angeschlossen ist.

2. Wasserleitung nach Patentanspruch 1, **dadurch gekennzeichnet, dass** die zweite Unterleitung (5) von der entsprechenden Kapsel (7) ausgeht und sich direkt mit der Kapsel (6) der ersten Unterleitung (4) verbindet, welche die sterile Flüssigkeit zuführt.

3. Wasserleitung nach Patentanspruch 1 oder 2, **dadurch gekennzeichnet, dass** jede Kapsel (6, 7) an dem oberen Ende der Kanüle (10, 11) mit einem Ventil (13, 14) versehen ist, das zusammen mit den Steuermitteln (12) wirkt, um zu erlauben, dass von den Flüssigkeiten, wenn erforderlich, jeweils nur eine in die einzige Unterleitung (4) fliesst.

4. Wasserleitung nach Patentanspruch 1 oder 2, **dadurch gekennzeichnet, dass** die mit der zweiten Unterleitung (5) zum Zuführen der desinfizierenden/sterilisieren Flüssigkeit verbundene Kapsel (7) mit einer Leitung (15) in Form eines umgekehrten "L" versehen ist, angeschlossen an die zweite Unterleitung (5), um die Flüssigkeit in Richtung der anderen Kapsel (6) abzuleiten.

5. Wasserleitung nach Patentanspruch 2, **dadurch gekennzeichnet, dass** die mit der ersten Unterleitung (4) zum Zuführen der sterilen Verbraucherflüssigkeit verbundene Kapsel (6) mit einer Leitung (16) in Form eines "T" versehen ist, um es den Flüssigkeiten zu ermöglichen, abwechselnd in die Hauptleitung (1) zu fliessen.

6. Wasserleitung nach Patentanspruch 3, **dadurch gekennzeichnet, dass** jede Kapsel (6, 7) mit einem Absperrventil (13, 14) versehen ist, angeordnet an dem oberen Ende der entsprechenden Kanüle (10, 11).

7. Wasserleitung nach Patentanspruch 1, **dadurch gekennzeichnet, dass** die Mittel (12) zum Steuern der Zufuhr einer jeden Flüssigkeit eine Luftleitung (12a, 12b) enthalten, eingearbeitet in jede Kapsel (6, 7) und dazu bestimmt, durch eine Einheit (17) zum Erzeugen eines Druckes im Inneren der jeweiligen Behälter (8, 9) auf solche Weise aktiviert zu werden, dass das Zuführen der gewählten Flüssigkeit erlaubt ist.

8. Wasserleitung nach Patentanspruch 1, **dadurch gekennzeichnet, dass** die zweite Unterleitung (5), welche die beiden Kapseln (6, 7) miteinander verbindet, mit einem zweiten Absperrventil (18) versehen ist, dazu bestimmt, die zweite Unterleitung (5) zu verschliessen, während die sterile Flüssigkeit benutzt wird.

## Revendications

1. Une conduite d'eau pour l'apport de fluides de stérilisation et de fluides stériles d'utilisation dans un équipement dentaire, la conduite d'eau en question comprenant un conduit principal (1) alimentant un fluide de réseau à une pluralité de branches (2) qui sont raccordées à des pièces à main dentaires (3) correspondantes ; le conduit principal (1) étant raccordé, en amont des branches de raccordement (2), au moins à une première sous-branche (4) qui distribue un fluide stérile d'utilisation à la place du fluide de réseau, et à une seconde sous-branche (5) qui distribue un fluide de désinfection/stérilisation dans le conduit principal (1) et les branches de raccordement (2) ; les première et seconde sous-branches (4, 5) étant chacune pourvues d'un élément ou capsule (6, 7), utilisée pour la fixation d'un récipient (8, 9) contenant les fluides à alimenter, et d'un tube (10, 11), raccordé à la première ou à la seconde sous-branche (4, 5), pouvant être introduit dans le récipient (8, 9) et destiné à aspirer le fluide sur commande de moyens de commande (12) correspondants ; ladite conduite d'eau étant **caractérisée en ce que** la seconde sous-branche (5) qui alimente le fluide de désinfection/stérilisation part de la capsule (7) correspondante et se raccorde directement avec la première sous-branche (4) qui alimente le fluide stérile, juste en aval du tube (10) correspondant, de manière à permettre aux moyens de commande (12) d'alimenter en alternance le fluide de désinfection/stérilisation et le fluide stérile le long d'une seule première sous-branche (4) raccordée au conduit principal (1).

2. La conduite d'eau selon la revendication 1, **caractérisée en ce que** la seconde sous-branche (5) part de la capsule (7) correspondante et se raccorde directement avec la capsule (6) de la première sous-branche (4) qui alimente le fluide stérile.

3. La conduite d'eau selon la revendication 1 ou 2, **caractérisée en ce que** chaque capsule (6, 7) est pourvue, au niveau de l'extrémité supérieure du tube (10, 11), d'une soupape (13, 14) qui agit en association avec les moyens de commande (12) pour permettre l'afflux des fluides, un seul à la fois, dans la seule sous-branche (4) lorsque cela est nécessaire.

4. La conduite d'eau selon la revendication 1 ou 2, **caractérisée en ce que** la capsule (7) associée à la seconde sous-branche (5) pour l'alimentation du fluide de désinfection/stérilisation est pourvue d'un conduit en « L » retourné (15) raccordé à la seconde sous-branche (5) elle-même pour dévier le fluide vers l'autre capsule (6).

5. La conduite d'eau selon la revendication 2, **caractérisée en ce que** la capsule (6) associée à la première sous-branche (4) pour l'alimentation du fluide stérile d'utilisation est pourvue d'un conduit en « T » (16) pour permettre l'afflux des fluides en alternance dans le conduit principal (1).

6. La conduite d'eau selon la revendication 3, **caractérisée en ce que** chaque capsule (6, 7) est pourvue d'un clapet anti-retour (13, 14) situé au niveau de l'extrémité supérieure du tube (10, 11) correspondant.

7. La conduite d'eau selon la revendication 1, **caractérisée en ce que** les moyens (12) de commande de l'alimentation de chaque fluide comprennent un conduit d'air (12a, 12b) réalisé dans chaque capsule (6, 7) et destiné à être activé par une unité (17) pour générer une pression à l'intérieur du récipient (8, 9) respectif afin de permettre l'alimentation du fluide sélectionné.

8. La conduite d'eau selon la revendication 1, **caractérisée en ce que** la seconde sous-branche (5) raccordant les deux capsules (6, 7) est pourvue d'une seconde soupape (18) de coupure, destinée à fermer la seconde sous-branche (5) elle-même quand le fluide stérile est utilisé.
